# EUROPEAN PATENT SPECIFICATION

(11) **EP 2 194 902 B1**
(45) Date of publication and mention of the grant of the patent: **15.01.2014**
(21) Application number: 08831322.6
(22) Date of filing: 16.09.2008
(51) Int. Cl.: A61B 18/18, A61B 18/12, A61B 18/14, H01Q 11/08

(54) **A RADIO FREQUENCY ENERGY TRANSMISSION DEVICE FOR THE ABLATION OF BIOLOGICAL TISSUES**
HOCHFREQUENZ-ENERGIEÜBERTRAGUNGSGERÄT ZUR ABLATION VON BIOLOGISCHEN GEWEBEN
DISPOSITIF DE TRANSMISSION D'ÉNERGIE HAUTE FRÉQUENCE POUR L'ABLATION DE TISSUS BIOLOGIQUES

(30) Priority: 20.09.2007 US 858736
(43) Date of publication of application: 16.06.2010
(73) Proprietor: Medwaves, Inc., San Diego, California 92127 (US)
(72) Inventor: ORMSBY, Theodore C., Escondido California 92029 (US); LEUNG, George L., San Diego California 92128 (US); LAW, Ming Fan, San Diego California 92129 (US)
(74) Representative: Purdy, Hugh Barry
(86) International application number: PCT/US2008/076523
(87) International publication number: WO 2009/039093

(56) References cited:
- WO-A1-2007/024878
- GB-A- 2 403 148
- US-A- 4 674 499
- US-A- 5 405 346
- US-A- 5 693 082
- US-B1- 6 287 302
- US-B1- 6 663 625
- US-B2- 7 004 938
- US-B2- 7 004 938

## Description

### Background

### 1. Field of the Invention

The invention relates to a radio frequency energy transmission device according to the preamble of claim 1 and according to the preamble of claim 5.
The present invention generally relates to medical devices, which are used for the irradiation of biological tissues, such as devices for the ablation of biological tissues, and more particularly to a radio frequency energy transmission device for such devices.

A radio frequency energy transmission device of the initially mentioned type is known for example from US 5 405 346 A.

A further radio frequency energy transmission device is known for example from US 7 004 938 B2.

### 2. Related Art

Therapeutic issue ablation systems apply energy to a biological ablation tissue site via different energy exchange means, such as heat conduction and irradiation. These systems may employ various energy modes, such as radiofrequency, ultrasound, laser, cryogenic, and the like. Within the radio frequency (RF) range, certain microwave ablation systems are used to destroy or ablate biological tissues. In one application, a microwave ablation system is used to ablate cardiac tissues that cause irregular heartbeats or arrhythmia, avoiding the need for more risky and invasive open heart surgery. In such an application, an ablation member such as an RF antenna is incorporated as part of a catheter. The catheter is passed through the vein for access to the atrium. Within the atrium, the RF antenna is positioned at the desired location where ablation is applied.

Microwave ablation systems can also be used in treatment of other biological sites such as arteries, organs and body vessels. As an example, a microwave ablation system is used to ablate tumors in the lungs, liver, kidney or other areas of the body.

These surgical and therapeutic applications require an efficient system for the transmission of radio frequency energy to the ablating member for the delivery of energy to the target tissue site.

### Summary

The present invention provides an innovative radio frequency energy transmission device for the ablation of biological tissues in body areas such as the heart, liver, and the like. The embodiments described herein provide a new conductive hollow coaxial cable device with a central lumen for use in a radio frequency based tissue ablation system.

The invention provides a radio frequency energy transmission device according to claim 1.

Other features and advantages of the present invention will become more readily apparent to those of ordinary skill in the art after reviewing the following detailed description and accompanying drawings.

### Brief Description of the Drawings

**Figure 1** is a schematic block diagram, partially broken away, illustrating one embodiment of radio frequency energy transmission device for the ablation of biological tissues;
**Figure 2** is a longitudinal cross-sectional view of a first embodiment of a hollow conductive coaxial cable for the device of Figure 1;
**Figure 3** is a cross-section taken on the lines 3-3 of Figure 2;
**Figure 4** is a cross-section taken on the lines 4-4 of Figure 2;
**Figure 5-1** is a partial isometric sectional view of a modified hollow conductive coaxial cable in which a dielectric layer is disposed between the inner and the outer electrical conductors of the cable;
**Figure 5-2** is a selected cross-sectional view of the modified hollow conductive coaxial cable shown in Fig. 5-1;
**Figure 5-3** is a cross-sectional view of another embodiment of the hollow conductive coaxial cable with two separate dielectric layers disposed between the inner and outer electrical conductors;
**Figure 5-4** is a cross-sectional view of a further alternative embodiment of the hollow conductive coaxial cable illustrating a plurality of dielectric layers disposed between the inner and outer electrical conductors;
**Figure 6-1** is a cross-sectional view of another variation of embodiment of the hollow conductive coaxial cable with an alternative dielectric layer disposed between the inner and outer electrical conductors;
**Figure 6-2** is a cross-sectional view of the dielectric material for use in the embodiment illustrated in Figure 6-1;
**Figure 6-3** is a partial isometric sectional view of the dielectric material for use in the illustrated in Figures 6-1 and 6-2;
**Figure 7-1** is a cross-sectional view of another embodiment of the dielectric material for placement between the inner and outer electrical conductors;
**Figure 7-2** is a partial isometric sectional view of the dielectric material for use in the embodiment illustrated in Figure 7-1;
**Figure 7-3** is a cross-sectional view of a further alternative embodiment of the dielectric material for placement between the inner and outer electrical conductors; and
**Figure 7-4** is a partial isometric sectional view of the dielectric material for use in the embodiment illustrated in Figure 7-3.

### Detailed Description

The present invention provides an innovative radio frequency energy transmission device, which incorporates a hollow coaxial cable for conducting radio frequency (RF) energy, particularly microwave energy, for the ablation of biological tissues. The hollow cable has a proximal end and a distal end and comprises coaxial inner and outer conductors. The inner conductor has an elongated electrically conductive tubular member with a hollow, axially extending lumen. The outer conductor has an elongated electrically conductive tubular member, which is arranged in a substantially coaxial relationship over the inner conductor. A dielectric medium is selectively disposed between the inner and outer conductors. An ablating member which delivers radio frequency energy, particularly microwave energy, at the distal end portion of the cable. The hollow conductive coaxial cable is adapted to connect with an RF signal generator at a proximal end and delivers the RF energy, particularly microwave energy to an ablation member mounted at a distal end portion.

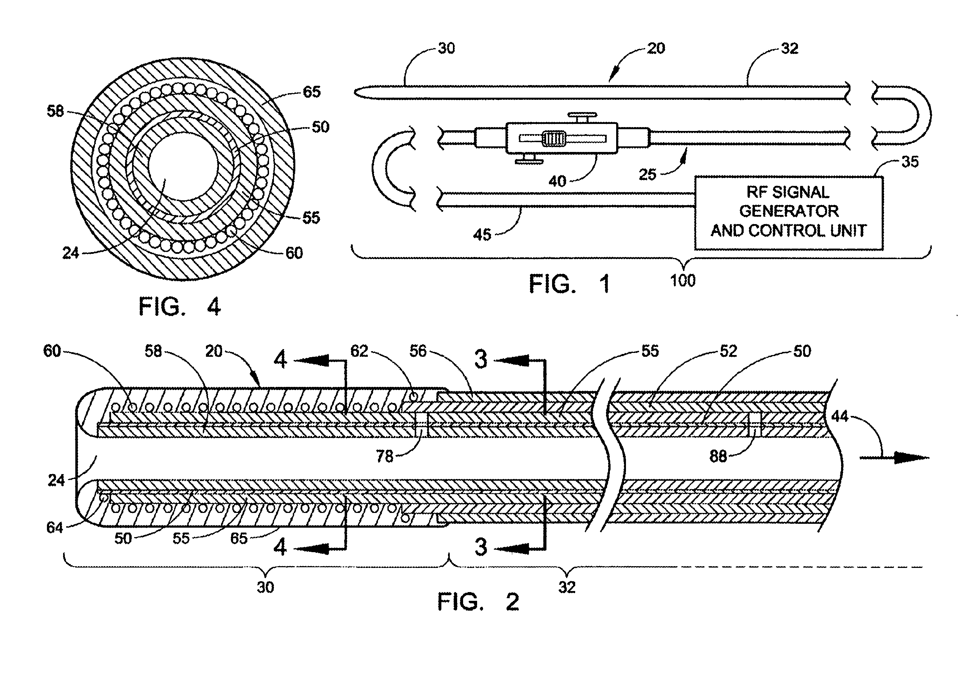

Figures 1 - 3 illustrate a radio frequency energy transmission (RF) energy ablation system 100, which comprises an elongated coaxial cable device 20 adapted for placement adjacent to or within a biological tissue site and/or a body vessel of a patient and an ablation device 60, such as an RF antenna, for delivering electromagnetic energy to the treatment site, as described in more detail below.

The coaxial cable device 20 has a flexible, elongated tubular body 32 having a proximal end portion 25 and a distal end portion 30. Located at the proximal end portion of the coaxial cable device is a handle unit 40, which contains steering and positioning controls (not illustrated) for the coaxial cable device. An RF signal generator and system control unit or system 35 is connected to the proximal end of the coaxial cable device by cable 45, and is electrically coupled to the ablation device 60 through the coaxial cable, as described in more detail below. The RF signal generator and control unit for controlling the RF signal delivered to the ablation device may be as described in US 2006287649

The structure of one embodiment of the coaxial cable device 20 is illustrated in more detail in Figures 2 to 4. The length and diameters of coaxial cable device 20 are adapted as required to suit the particular medical procedure, as is known in the medical art. Coaxial device 20 is generally tubular and has a multi-layer construction with a central bore or lumen 24 extending along its length. The distal end 30 of the lumen 24 may be close as illustrated in Figures 2 or it may be open in other embodiments, for example as described and shown in U.S. Patent No. 6,663,625.

The coaxial cable device 20 comprises a first or inner electrically conductive tubular member or conductor 50 having a proximal end portion and a distal end portion. Inner conductor 50 is constructed of an elongated electrically conductive tubular member having a hollow lumen 24. An outer conductor 52, also made of an elongated electrically conductive tubular member, is arranged in a substantially coaxial relationship over at least a portion of length of the inner conductor 50. This arrangement defines a space 54 between the walls of the inner conductor 50 and the outer conductor 52.

An ablation device 60 is located at the distal end portion 30 of the coaxial cable device 20 and is electrically coupled to both the outer coaxial conductor 52 at contact point 62 and to the inner conductor 50 at contact point 64. In turn, the inner conductor and the second or outer conductor are electrically coupled to the RF energy source in unit 35. In the illustrated embodiment, the ablation device 60 comprises a helical coil wound around the outer circumferential surface of the coaxial cable device and extending from the end portion of the outer conductor 52 up to the distal end portion or tip of the device 20. The helical coil 60 is coated with an outer coating layer 65 of dielectric material such as a polymeric dielectric encapsulant which protects the structural integrity of the coil and also shields it from the surrounding biological environment. In alternative embodiments, other forms of ablation devices or radio frequency antennas may be used in place of the helical coil antenna 60, such as a monopole bead antenna or a pair of spaced electrically conductive microstrips disposed at the distal end portion of the coaxial cable device, as described in U.S. Patent No. 6,663,625 referenced above. The RF antenna 60 includes an electrically conductive material or wire strip that is wound in a helical fashion to form a helical coil. The appropriate diameter, pitch and length of the coil winding, and the selection of the conductive material or wire strip are a matter of choice, which can vary according to the particular procedure requirements as known in the art. Thus these design elements and considerations are not detailed here.

As shown in Figures 1 - 3, a dielectric medium 53 is provided in space 54 to impede electrical conduction between the inner conductor 50 and outer conductor 52. Selectively, the dielectric is formed of a dielectric layer 55, which substantially fills the space 54 between the inner conductor 50 and outer conduction 52, being on unfilled space. Vacuum, exhibiting dielectric property, is introduced by means of a vacuum source configured in fluid communication with space 54, as discussed in more detail below.

An outer jacket or casing 56 encases the outer conductor 52 along the length of the coaxial cable device up to the distal end portion 30. The outer casing 56 is generally constructed of a polymer material that is bio-compatible within the body vessel environment. Examples of such materials include thermoplastic elastomer material such as Pebax® available from Autochem Germany, polyethylene, polyurethane, polyester, polyimide, polyamide, and the like, with varying degrees of radiopacity, hardness, and elasticity.

The tubular body of the coaxial cable device 20 may be formed with a plurality of segments using one or more of the aforementioned materials or equivalents, such that the device 20 is progressively more flexible towards its distal end. The segments may be joined together by thermal bonding, butt joints, or adhesive bonding. Braiding reinforcement may be provided to the surface of the tubular body to attain a desirable level of stiffness and torsional strength for the device to advance and negotiate through the body vessel of the patient, while still allowing the distal end portion to be bent when needed. The distal end portion 30 may be of a softer polymer compound than the remainder of the body, with little or no braiding or reinforcement, to provide the desired flexibility for distal deflection and shaping of the apparatus.

In one embodiment, inner conductor 50 may be made of a flexible braided wire construction or thin film electrically conductive material. An inner liner or sleeve 58 of flexible dielectric material may be provided inside conductor 50 to surround the hollow central bore or lumen 24. The outer conductor 52 may be of a braided wire construction or may be a thin film electrically conductive material or the like. The sleeve 58, the inner conductor 50, and the dielectric layer 55 extend from handle unit 40 through the distal end portion of the coaxial cable device, while the outer conductor 52 and outer casing 56 extend from the handle unit 40 and terminate short of the distal end of the device, with the outer conductor projecting a short distance beyond the distal end of the outer casing, as seen in Figure 2.

The RF antenna 60 is adapted to receive and radiate electromagnetic energy from a source of radio frequency energy (not shown) in unit 35. An example of suitable spectrum of radio frequency is that of the microwave frequency ranging from approximately 300 MHz and up. The RF antenna 60 imparts substantially uniformly distributed electromagnetic field energy transmitted by the helical coil. The power of the electromagnetic field transmitted is substantially normal to the longitudinal axis of the RF antenna, and a uniform energy field is produced circularly about and bounded by the antenna. The energy delivered for the ablation is substantially uniformly distributed along the antenna, which is independent of the contact between the antenna and the tissue to be ablated.

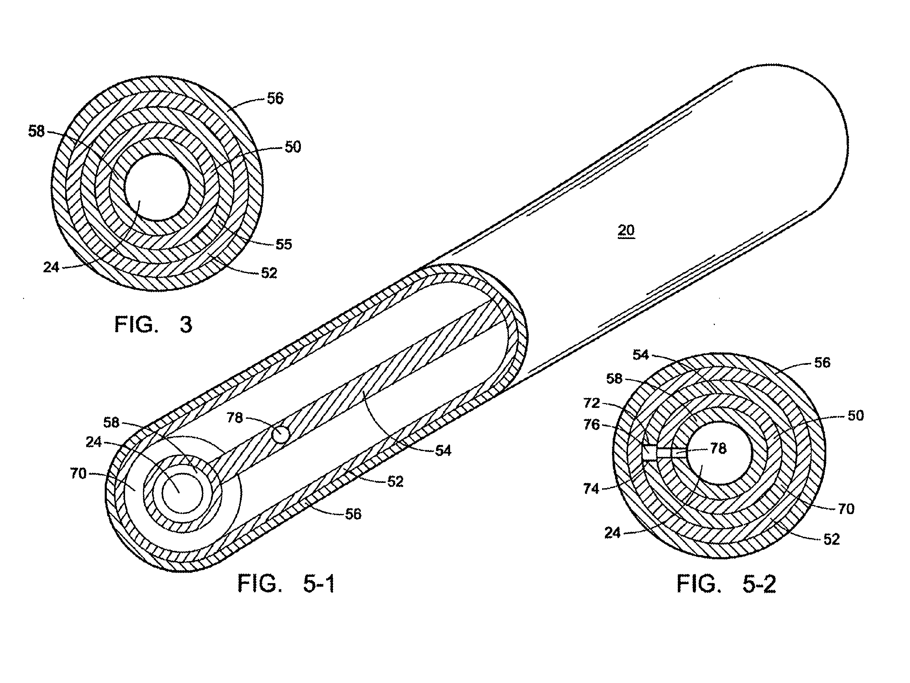

Figures 5-1 and 5-2 show another embodiment, which incorporates an alternative dielectric medium configuration. Like reference numerals in Figures 5-1 and 5-2 are used for like parts in other figures as appropriate. In this embodiment, the dielectric medium 53 is constructed of a dielectric layer 70, which is disposed in the space 54 between the inner and outer conductors to wrapping around the inner conductor 50. A gap 76 is provided between the longitudinal peripheral edges 72 and 74 of the dielectric layer 70. Gap 76 extends along at least a portion of the length of the coaxial cable and is generally oriented in parallel with the axis of the cable, though other directional alignment can be provided. Additionally, the peripheral edges 72 and 74 of the dielectric layer 70 may be joined in selected locations to define a plurality of voids along the seam of the peripheral edges in the space 54 between the inner conductor 50 and outer conductor 52.

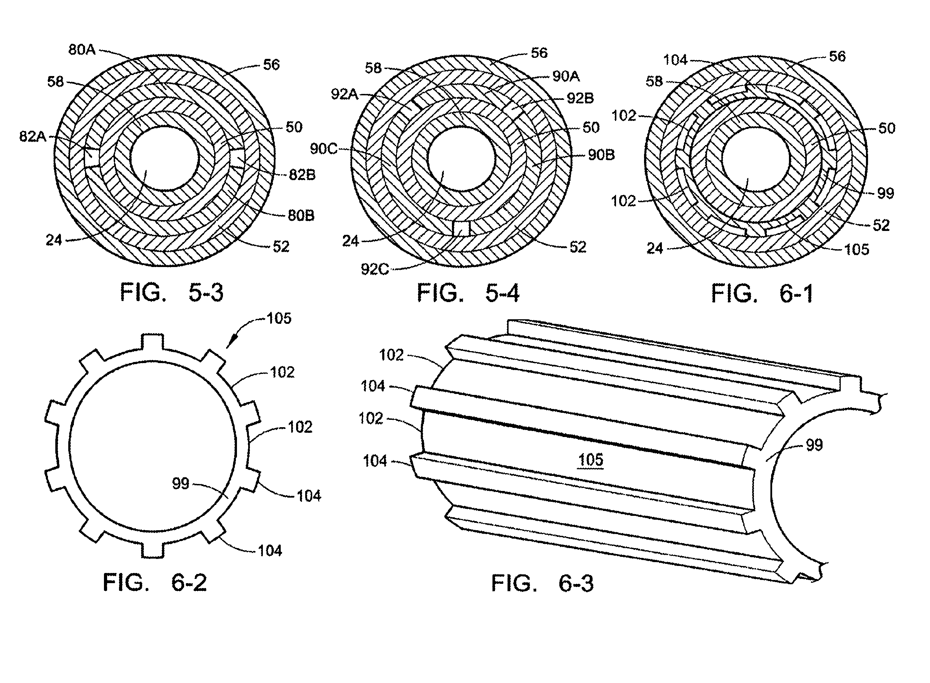

Figures 5-3 and 5-4 show the cross-sectional views of additional embodiments of the hollow conductive coaxial cable wherein two or more separate dielectric layers are disposed between the inner and outer electrical conductors. Figure 5-3 illustrates a configuration where two pieces of dielectric layers 80A, 80B are provided in space 54. The two dielectric layers are separated by gaps 82A and 82B. Similar to the embodiment shown in Figures 5-1 and 5-2, gaps 82A and 82B each extend along at least a portion of the length of the coaxial cable in a generally parallel direction with the axis of the inner and outer conductors, though other directional alignment can be provided. Gaps 80A and 80B thus provide elongated channels between the dielectric layers along the length of the coaxial cable in the space 54 between the inner and outer conductors.

In Figure 5-4, three pieces of dielectric layers 90A, 90B, 90C are provided in space 54. The dielectric layers are separated by gaps 92A, 92B and 93C. Similar to the embodiments shown in Figures 5-1 - 5-4, the orientation of the gaps 92A, 92B and 92C extends in a generally parallel direction with the axis of the inner and outer conductors, though other directional alignment can be provided.

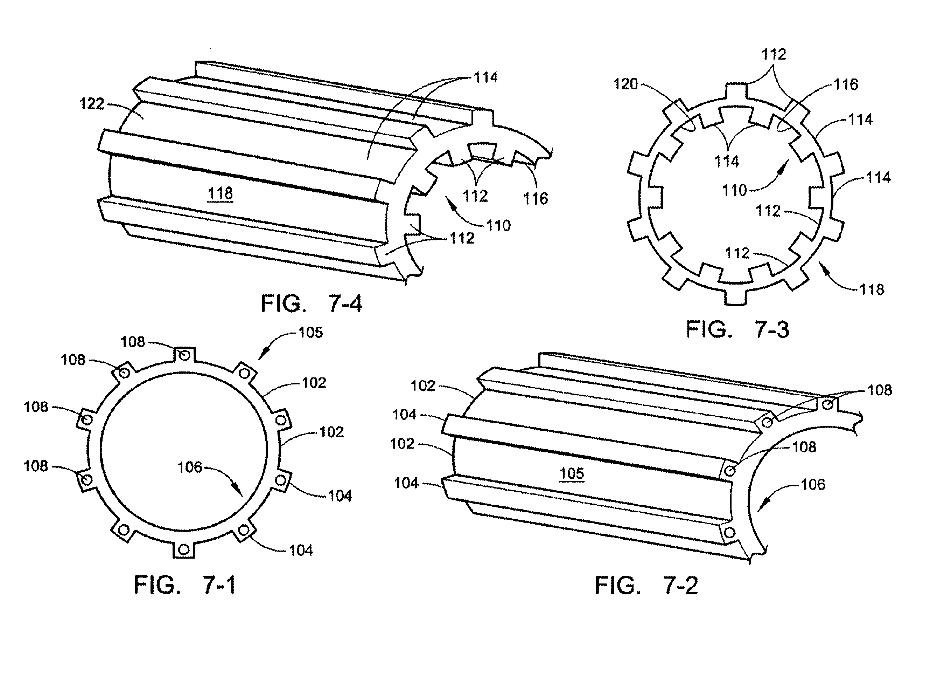

Figure 6-1 shows a further embodiment, which incorporates an alternative dielectric material configuration. A dielectric layer 99 is provided with one or more or surface recesses 102 and is disposed between the inner conductor 50 and the outer conductor 52. As exemplified by the embodiment illustrated in the cross-sectional and partial isometric sectional view of Figures 6-2 and 6-3, the recesses 102 are formed between the elongated spines or upraised ridges 104, which extend in a substantially parallel relationship with the axis of the inner and outer conductors. This embodiment defines at least one channel extending between the distal and the proximal end portions of the coaxial cable. As shown in the embodiment illustrated in Figures 6-1, 6-2 and 6-3, spines 104 are arranged in an equal-angular relationship about the axis of the coaxial cable. Spines 104 can be formed as part of the dielectric layer 99. Alternatively, they can be formed as separate elongated strips and affixed on the surface 105 of the dielectric layer 99. Further, spines 104 can assume various cross-sectional profiles, which are not limited to those as shown in Figures 6-1, 6-2, 6-3, 7-1, 7-2, 7-3 and 7-4.

Optionally, the recesses 102 can be formed and oriented to extend in a spinal fashion relative to the axis of the inner and outer conductors, thus defining one or more spinal channels or passageways in space 54 (not shown) between the inner conductor 50 and the outer conductor 52. As a further alternative design, the lineal recesses can be formed in an intersecting crisscross fashion on either one side or both sides (not shown) of the dielectric layer 100 disposed between the inner conductor 50 and outer conductor 52. Further, in lieu of indentation, lineal or otherwise, formed on the surface of dielectric material, the recesses may be in the form of perforations or voids (not shown).

Figures 7-1 and 7-2 illustrate a dielectric layer configuration 106 in which at least one internal passageway 108 is selectively formed in the elongated ridges 104 and extending along the length of the ridges 104 to follow the length of the coaxial cable. This alternative dielectric configuration provides at least one open channel 102 between the elongated spines and at least one internal passageway 108 extending between the distal and the proximal end portions of the coaxial cable.

Figures 7-3 and 7-4 illustrate another variation of the embodiment of the present invention where an alternative dielectric layer 110 configured in the space 54 between the inner conductor 50 and the outer conductor 52 is provided with one or more or surface recesses 114 on both surfaces of the dielectric layer 110. Similar to the embodiments described above, the recesses are formed between elongated ridges 112 which extend on the inner surface 116 and outer surface 118 of the dielectric layer 110 along the longitudinal direction of the coaxial cable. This embodiment provides elongated inner channels 120 and outer channels 122 between the distal portion and the proximal portion of the coaxial cable from the distal portion to the proximal portion of the coaxial cable.

In the embodiments presented herein, the inner conductor 50 and outer conductor 52 are configured in a substantially coaxial relationship in which the walls between the conductors define a space 54 extending in the length of the coaxial cable. As discussed above, the space 54 is configured to interpose dielectricity, which impedes electrical conduction between the inner and outer conductors, effected with the introduction of a vacuum.

Access openings are formed on the distal portion and/or proximal portion of the coaxial cable to provide communication between space 54 and hollow lumen 24. As illustrated in Figures 5-1 and 5-2, the access opening 78 at the distal portion and access opening 88 at the proximal portion are selectively formed on the inner conductor 50 and inner liner 58. Such a feature provides an enhanced versatility to the ablation device to enable access to the space between the inner cable and the outer cable. It also provides an additional means to facilitate the introduction of vacuum, placement of devices and instruments and dispensing of medication, such as drugs, saline and sterile water to the patient in support of the ablation operation.

The outer dimensions of the body of the coaxial cable device in each of the above embodiments may be adapted as required to suit the particular medical procedure, as is well known in the medical art. In one embodiment, the device is used to ablate cardiac tissue. However, the device may be used to ablate other types of body tissue in different organs, both internal and external to the body. The tubular body of the coaxial cable device may be generally constructed of a polymer material which is bio-compatible with the body vessel environment.

In each of the above embodiments, the ablation device or RF antenna is adapted to receive and radiate electromagnetic energy in order to treat a selected biological tissue site by changing a property of the biological tissue at the site. An example of a suitable spectrum of radio frequency energy for use in tissue ablation is that of the microwave frequency range above 300 MHz. The RF antenna is capable of applying substantially uniformly distributed electromagnetic field energy along the RF antenna in a direction substantially normal to the longitudinal axis of antenna 60. The elongated, flexible coaxial cable device connected to an RF source and control unit at its proximal end extends to a distal end portion at which the RF antenna is mounted. The coaxial cable device in each of the foregoing embodiments has coaxial inner and outer conductors extending from its proximal end and separated by a dielectric medium, and a central lumen or bore inside the inner conductor extends the length of the coaxial cable device and can be used to accommodate conductor wires which are connected to ECG electrodes, temperature sensors, or the like, as well as a suitable shaping or steering mechanism for controlling the shape or deflection of the distal end portion of the coaxial cable device in which the RF antenna is located.

## Claims

1. A radio frequency energy transmission device (100) for the ablation of biological tissues, comprising: a hollow coaxial electrical cable (20) having a proximal end portion (25) and a distal end portion (30) and comprising (i) an inner conductor (50) comprising an elongated electrically conductive tubular member having a hollow lumen (24); and (ii) an outer conductor (52) comprising an elongated electrically conductive tubular member disposed in a coaxial relationship over at least a portion of the inner conductor (50) and defining a space (54) between the walls of the inner conductor (50) and the outer conductor (52) wherein a dielectric medium (53) is interposed; an ablating member (60) electrically coupled to the hollow coaxial cable (20), which transmits radio frequency energy to the biological tissue; and **characterized in that** the space (54) between the inner and the outer conductors (50,52) is in fluidic communication with a source of vacuum, wherein the space (54) extends between the distal and proximal end portions of the coaxial cable.

## Patentansprüche

1. Radiofrequenz-Energieübertragungsvorrichtung (100) für die Ablation von biologischen Geweben, die Folgendes umfasst: ein hohles elektrisches Koaxialkabel (20) mit einem proximalen Endabschnitt (25) und einem distalen Endabschnitt (30) und umfassend (i) einen inneren Leiter (50), umfassend ein langgestrecktes elektrisch leitfähiges röhrenförmiges Element mit einem hohlen Lumen (24); und (ii) einen äußeren Leiter (52), umfassend ein langgestrecktes elektrisch leitfähiges röhrenförmiges Element, das in einer koaxialen Beziehung über mindestens einem Abschnitt des inneren Leiters (50) angeordnet ist und einen Zwischenraum (54) zwischen den Wänden des inneren Leiters (50) und des äußeren Leiters (52) definiert, in dem ein dielektrisches Medium (53) angeordnet ist; wobei ein Ablationselement (60) elektrisch an das hohle Koaxialkabel (20) gekoppelt ist, das Radiofrequenzenergie an das biologische Gewebe überträgt; und **dadurch gekennzeichnet, dass** der Zwischenraum (54) zwischen dem inneren und dem äußeren Leiter (50, 52) in Fluidverbindung mit einer Quelle von Unterdruck steht, wobei sich der Zwischenraum (54) zwischen dem distalen und dem proximalen Endabschnitt des Koaxialkabels erstreckt.

## Revendications

1. Dispositif de transmission d'énergie de fréquence radioélectrique (100) pour l'ablation de tissus biologiques, comprenant : un câble électrique coaxial creux (20) ayant une partie d'extrémité proximale (25) et une partie d'extrémité distale (30) et comprenant (i) un conducteur interne (50) comprenant un élément tubulaire électriquement conducteur allongé ayant une lumière creuse (24) ; et (ii) un conducteur externe (52) comprenant un élément tubulaire électriquement conducteur allongé disposé selon une relation coaxiale par-dessus au moins une partie du conducteur interne (50) et définissant un espace (54) entre les parois du conducteur interne (50) et du conducteur externe (52) dans lequel un milieu diélectrique (53) est interposé ; un élément d'ablation (60) couplé électriquement au câble coaxial creux (20), lequel transmet une énergie de fréquence radioélectrique au tissu biologique ; et **caractérisé en ce que** l'espace (54) entre les conducteurs interne et externe (50, 52) est en communication fluidique avec une source de vide, l'espace (54) s'étendant entre les parties d'extrémité distale et proximale du câble coaxial.
